# EUROPÄISCHE PATENTSCHRIFT

(11) **EP 1 446 668 B1**
(45) Veröffentlichungstag und Bekanntmachung des Hinweises auf die Patenterteilung: **11.10.2006**
(21) Anmeldenummer: 02787585.5
(22) Anmeldetag: 07.11.2002
(51) Int. Cl.: G01N 35/00, G01N 33/543, B03C 1/04

(54) **VORRICHTUNG UND VERFAHREN ZUM BEHANDELN VON MAGNETPARTIKELN**
DEVICE AND METHOD FOR TREATING MAGNETIC PARTICLES
DISPOSITIF ET PROCEDE DE TRAITEMENT DE PARTICULES MAGNETIQUES

(30) Priorität: 19.11.2001 DE 10156790
(43) Veröffentlichungstag der Anmeldung: 18.08.2004
(73) Patentinhaber: chemagen Biopolymer-Technologie AG, 52499 Baesweiler (DE)
(72) Erfinder: à BRASSARD, Lothar, 52525 Heinsberg (DE)
(74) Vertreter: Flaccus, Rolf-Dieter
(86) Internationale Anmeldenummer: PCT/EP2002/012411
(87) Internationale Veröffentlichungsnummer: WO 2003/044537

(56) Entgegenhaltungen:
- EP-A- 0 589 636
- EP-A- 0 905 520
- DE-A- 10 063 984
- US-A- 5 705 062
- US-A- 6 150 182
- US-B1- 6 187 270

## Beschreibung

Die Erfindung bezieht sich auf Vorrichtungen und Verfahren zum Behandeln von magnetisierbaren oder magnetisch anziehbaren Partikeln (Magnetpartikeln). Sie bezieht sich insbesondere auf Magnetpartikel, die spezifische Bindungseigenschaften für bestimmte Substanzen aufweisen und zur Abtrennung dieser Substanzen aus komplexen Gemischen verwendet werden.

Im Stand der Technik wurden folgende Vorrichtungen und Verfahren beschrieben:
EP 0 589 636 A1 offenbart eine Vorrichtung zur Behandlung von magnetischen Partikeln umfassend ein Gefäß, das zwischen zwei in vertikaler Richtung bewegbaren Magneten angeordnet ist. Dabei bewegen sich die Magnete gemeinsam.
US 5,705,062 offenbart eine Vorrichtung mit einer starren Magnetanordnung, deren Polarachsen jedoch um 45° zur Längsachse des Reaktionsgefäßes gekippt sind. Das Reaktionsgefäß selber wird in einer Drehbewegung in die Magnetanordung gebracht.
US 6,150,182 offenbart eine Vorrichtung mit einem in vertikaler Richtung beweglichen Ringmagneten. Dort nutzt man durch Drehung des Reaktionsgefäßes zusätzlich die Zentrifugalkraft, um die Partikel an der Gefäßwand zu immobilisieren.
US 6,187,270 offenbart eine Vorrichtung, in der ein Magnet seitlich zum Reaktionsgefäß angeordnet wird und vertikal verschoben werden kann.
Ein Resuspendieren in einem neuen Lösungsmittel oder Separation der magnetischen Partikel wird bei allen oben genannten Anmeldungen nach Entfernung der Magnete durch Bewegung des Reaktionsgefäßes oder Spülung bewirkt.
EP 0 905 520 A1 offenbart eine Vorrichtung in der das Waschen und die Separation der magnetischen Partikel in mehreren Stufen mit der Bewegung der Partikel durch das Lösungsmittel mittels einer rotierenden Magnetanordnung realisiert wird.

Die Methode der Abtrennung von Substanzen unter Verwendung von Magnetpartikeln ist sehr vielseitig einsetzbar, da derartige Magnetpartikel mit den unterschiedlichsten Bindungseigenschaften ausgestattet werden können. Beispielsweise können sie zur sequenzspezifischen Isolierung von Nukleinsäuren oder bei immunologischen Assays, die auf Antigen-Antikörper-Bindung beruhen, eingesetzt werden.
Verfahren, die auf der magnetischen Abtrennung unter Verwendung von spezifisch bindenden, magnetisch anziehbaren Partikeln beruhen, erlangen im Bereich der Probenvorbereitung für diagnostische oder analytische Untersuchungen zunehmende Bedeutung. Dies gilt insbesondere für automatisierte Verfahren, da auf diese Weise eine große Anzahl von Proben innerhalb kurzer Zeit analysiert werden können. Dadurch werden die Voraussetzungen für ein effizientes Screening mit einem hohen Proben-Durchsatz geschaffen. Dies ist beispielsweise für Anwendungen bei molekulargenetischen Studien oder auf dem Gebiet der genetischen Diagnostik von enormer Bedeutung, da eine rein manuelle Handhabung von sehr großen Probenzahlen praktisch nicht zu bewältigen ist.

Das Grundprinzip der magnetischen Abtrennung von Substanzen aus komplexen Gemischen ist denkbar einfach. Magnetische Partikel (magnetisierbare bzw. magnetisch anziehbare Partikel) werden in spezifischer Weise für den beabsichtigten Separationsprozeß funktionalisiert, d. h. sie werden durch chemische Behandlung mit spezifischen Bindungseigenschaften für die abzutrennenden Zielsubstanzen ausgestattet. Für bestimmte häufig auftretende Anwendungszwecke sind derartige funktionalisierte Magnetpartikel auch kommerziell erhältlich. Die Größe solcher Magnetpartikel liegt im allgemeinen im Größenbereich von ca. 0,05 bis 500 µm.

In einem ersten Schritt ("Bindungs-Schritt") des bekannten Trennverfahrens werden die genannten funktionalisierten Magnetpartikel in einem Reaktionsgefäß zu einem aufzureinigenden (Ausgangs-) Gemisch hinzugegeben, das die zielsubstanz(en) in einer Flüssigkeit enthält, welche die Bindung der Zielsubstanz-Moleküle an die Magnetpartikel begünstigt (Bindungspuffer bzw. Bindungs-Flüssigkeit). Dadurch kommt es zu einer selektiven Bindung der im Gemisch vorhandenen Zielsubstanz(en) an den/die Magnetpartikel (= Bindungsschritt). Da die Bindungsreaktion in der Regel zeitabhängig verläuft, muß der Bindungs-Schritt über einen bestimmten Zeitraum hinweg durchgeführt werden, um eine möglichst vollständige Bindung der vorhandenen Zielsubstanz-Moleküle an die Magnetpartikel zu erreichen. Problematisch ist dabei, daß die Magnetpartikel aufgrund der gravimetrischen Kräfte dazu neigen, zu sedimentieren. Dadurch wird die Effizienz der Bindung von Zielgruppen-Molekülen an die Magnetpartikel beeinträchtigt, vor allem aufgrund der eingeschränkten Diffusion.

Anschließend werden diese Magnetpartikel durch Anwendung magnetischer Kräfte bzw. eines Magnetfeldes, beispielsweise mittels eines Permanentmagneten, an einer Stelle der Reaktionsgefäß-Innenwand immobilisiert (reversibel) und bilden dort in der Regel ein "Pellet".

Nachfolgend wird der flüssige Überstand abgetrennt und verworfen, beispielsweise durch Absaugen oder Dekantieren. Da die Magnetpartikel auf die genannte Weise immobilisiert sind, wird weitgehend verhindert, daß diese Partikel mit dem Überstand abgetrennt werden.

Auf diesen Bindungsschritt folgt in der Regel ein (oder mehrere) Waschschritt(e), wobei das Pellet der Magnetpartikel in einer geeigneten Waschflüssigkeit resuspendiert und mit dieser vermischt wird, Die Waschflüssigkeit und die Bedingungen des Waschens werden so ausgewählt, daß die spezifische Bindung der Zielsubstanz an die Magnetpartikel nicht beeinträchtigt wird. Dadurch wird durch das Waschen eine Abtrennung unspezifisch gebundener Substanzen bewirkt und letztendlich die Reinheit der abzutrennenden Zielsubstanz erhöht. Wie zuvor für den Bindungsschritt beschrieben, wird auch nach dem/den Waschschritt(en) der Überstand (d. h. die Waschflüssigkeit) nach Immobilisieren der Magnetpartikel abgetrennt und verworfen.

In dem auf den/die Waschschritt(e) folgenden Elutionsschritt werden die als Pellet immobilisierten Magnetpartikel erneut resuspendiert. Dabei wird eine Elutionsflüssigkeit bzw. ein Elutionspuffer verwendet, der geeignet ist, die Bindung zwischen der/den Zielsubstanz(en) und den Magnetpartikeln zu lösen, so daß die Zielsubstanz-Moleküle von den Magnetpartikeln freigesetzt werden und mit der Elutionsflüssigkeit abgetrennt werden können. Während des Abtrennens der Elutionsflüssigkeit werden die Magnetpartikel immobilisiert, wie oben beschrieben.

Die Zusammensetzungen der bei diesen Trennverfahren eingesetzten Bindungs-, Wasch- und Elutionsflüssigkeiten sind dem Fachmann bekannt oder können im Einzelfall durch einfache Vorversuche ermittelt werden.

Der Vorgang des Resuspendierens der immobilisierten Magnetpartikel (Magnetpartikel-Pellet) hat einen entscheidenden Einfluß auf die Reinheit und Ausbeute der abzutrennenden Zielsubstanzen (das Separationsprodukt).
Wenn die Magnetpartikel während des Waschens nur ungenügend resuspendiert werden und Agglomerate von Partikeln zurückbleiben, hat dies zur Folge, daß das Waschen ineffizient ist und die beabsichtigte Abtrennung unspezifisch gebundener Verunreinigungen nicht oder in nicht ausreichendem Maße stattfindet. Die Reinheit der abgetrennten Zielsubstanzen bzw. des Separationsprodukts ist in diesem Fall von minderer Qualität.

Wenn die Magnetpartikel während des Elutionsschrittes nicht vollständig oder nur ungenügend resuspendiert werden, hat dies zur Folge, daß die Elution nur unvollständig von statten geht, so daß lediglich ein Teil der an die Magnetpartikel gebundenen Zielsubstanzen von den Partikeln gelöst wird, während ein anderer Teil daran gebunden bleibt und nicht in die Elutionsflüssigkeit übertritt. Dadurch wird die Ausbeute mehr oder weniger stark vermindert, was insbesondere dann nachteilig ist, wenn die abzutrennenden Zielsubstanzen nur in sehr geringen Konzentrationen in dem untersuchten Ausgangsgemisch enthalten sind.
Besonders ungünstig ist es, wenn infolge ungenügenden Resuspendierens während des Waschens und während des Eluierens sowohl die Reinheit als auch die Ausbeute des abzutrennenden Produkts vermindert werden.

Der Vorgang des Resuspendierens ist insbesondere dann problematisch, wenn der oben beschriebene Ablauf des Trennverfahrens mittels automatisierter vorrichtungen durchgeführt wird, die einen hohen Probendurchsatz ermöglichen sollen.

Das Resuspendieren wird zusätzlich dadurch erschwert, daß die Probenvolumina bzw. die verwendeten Reaktionsgefäße sehr klein sind (Miniaturisierung). Zudem soll die Resuspendierung innerhalb eines möglichst kurzen Zeitraums erfolgen, um einen hohen Probendurchsatz zu gewährleisten.

Das Resuspendieren kann beispielsweise dadurch bewirkt werden, daß die über dem Magnetpartikel-Pellet befindliche Flüssigkeit mehrfach auf- und abpipettiert wird, bis das Pellet zerfällt und die Partikel in der Flüssigkeit verteilt sind. Allerdings werden für diesen Vorgang zusätzliche Einmal-Pipettenspitzen verbraucht, was angesichts der hohen Probenzahlen zu deutlichen Mehrkosten führt.

Eine andere bekannte Möglichkeit besteht darin, sich bewegende (z. B. rotierende) Rühr-Elemente, wie z. B. Permanentmagnet-Stäbe, in die Reaktionsgefäße einzutauchen und auf diese Weise einen Zerfall des Pellets und eine Durchmischung und Resuspendierung herbeizuführen. Für sehr kleine Probenvolumina, wie sie bei Hochdurchsatz-Screeningverfahren verwendet werden, ist diese Methode des Resuspendierens nur bedingt geeignet, da das eintauchende Rühr-Element zusätzlichen Raum benötigt. Außerdem kann das Eintauchen das nachfolgende Herausnehmen der genannten Rühr-Elemente zu Verlusten an Probenmaterial oder zu Kontaminationen führen, sofern nicht entsprechende Gegenmaßnahmen getroffen werden.

Außerdem kann das Resuspendieren in bekannter Weise durch Vibrationen oder durch einen Schüttler bewirkt werden. Dies hat allerdings den Nachteil, daß zusätzliche Apparaturen benötigt werden (Kostenfaktor) und daß für diesen Vorgang zusätzliche Arbeitsschritte und Zeit benötigt werden, beispielsweise um die Proben in den Schüttler einzusetzen und danach wieder herauszunehmen. Zudem ist diese Methode insbesondere bei sehr kleinen Reaktionsvolumina nur wenig geeignet, um beispielsweise ein Pellet von Magnetpartikeln zu resuspendieren.

Es bestand deshalb die Aufgabe, Vorrichtungen und Verfahren zum Behandeln von Magnetpartikeln bereitzustellen, die bei magnetischen Trennverfahren der beschriebenen Art eine effizientere und schnellere Resuspendierung der pelletierten bzw. immobilisierten Magnetpartikel insbesondere bei den Wasch- und Elutionsschritten ermöglichen, und die eine effizientere Durchführung des Bindungsschrittes ermöglichen, wobei die oben erwähnten Nachteile sowie andere Nachteile bekannter Methoden vermieden werden sollen.

Die Lösung dieser Aufgaben wird durch Vorrichtungen gemäß Anspruch 1 sowie mit den in den Ansprüchen 13 und 14 beschriebenen Verfahren ermöglicht, sowie durch die in den abhängigen Unteransprüche beschriebenen bevorzugten Ausführungsformen dieser Vorrichtungen bzw. Verfahren.

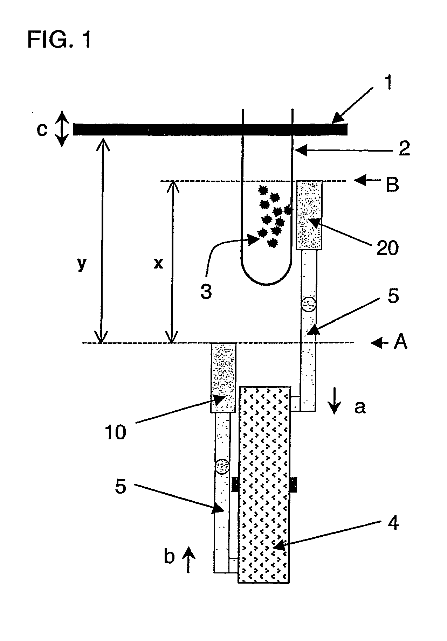

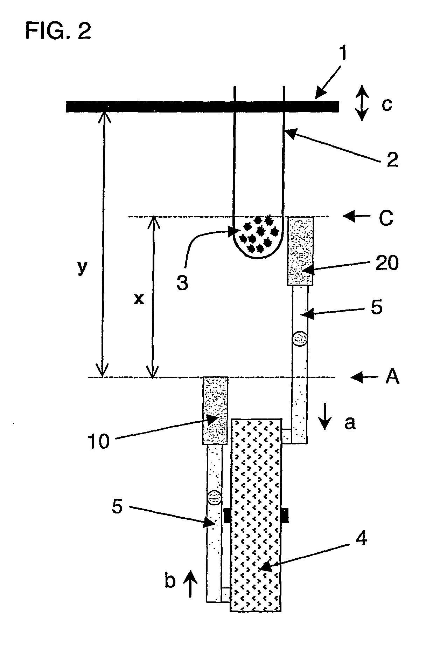

Die Erfindung wird nachfolgend anhand der in Fig. 1 und Fig. 2 gezeigten schematischen Darstellungen näher erläutert. Diese Zeichnungen sollen lediglich das Funktionsprinzip veranschaulichen und sollen keinesfalls als Einschränkung auf die dargestellten Ausführungsformen verstanden werden.
Fig. 1 bzw. Fig. 2 zeigen jeweils eine schematische (Längs-)Schnittdarstellung der erfindungsgemäßen Vorrichtungen.

Nach der vorliegenden Erfindung umfaßt eine Vorrichtung zum Behandeln von magnetisierbaren oder magnetisch anziehbaren, in einer Flüssigkeit vorliegenden Partikeln (3) eine Halterung (1) für ein oder mehrere Reaktionsgefäß(e) (2) mit der die Partikel (3) enthaltenden Flüssigkeit, mindestens zwei beweglich angeordnete Permanentmagnete (10,20) sowie eine Antriebseinheit (4) zum Bewegen der Permanentmagnete (10,20).

Die Permanentmagnete (10,20) sind paarweise und sich gegenüberliegend angeordnet und in vertikaler Richtung (Pfeile a, b) auf- und abwärts bewegbar.
Der Abstand zwischen den zwei Permanentmagneten (10,20) eines Paares ist so bemessen oder einstellbar, daß ein Reaktionsgefäß (2) zwischen diesen positioniert werden kann. Die erwähnte "gegenüberliegende Anordnung" schließt auch solche Anordnungen mit ein, bei denen sich die Magnete nicht exakt auf einer Linie (180°) gegenüberliegen; vielmehr kann dieser Winkel auch weniger als 180° betragen (ca. 180° bis ca. 120°), oder die beiden Permanentmagnete können seitlich versetzt zueinander angeordnet sein, bezogen auf das dazwischen angeordnete Reaktionsgefäß.

Die Vorrichtungen sind derartig aufgebaut oder steuerbar, daß die genannte Bewegung der Permanentmagneten (10,20) in der Weise erfolgt, daß jeder Permanentmagnet einer paarweisen Anordnung aus einer ersten Position oder Ausgangsposition (A), in der er sich im Abstand zu einem Reaktionsgefäß befindet und in der er im wesentlichen keinen Einfluß auf die genannten Partikel (3) ausübt (Magnet (10) in Fig. 1 und 2), in eine zweite oder in eine andere Position (Arbeitsposition) (B, C) gebracht werden kann, in der er sich seitlich neben dem betreffenden Reaktionsgefäß (2) befindet und in der er eine magnetische Kraft auf die Partikel (3) ausübt (Magnet (20) in Fig. 1 u. 2). Der Abstand x zwischen den beiden Positionen (A, B; gestrichelte Linien in Fig. 1 u. 2) ist derartig eingestellt, daß die magnetische Kraft des unten stehenden Magneten (Position A) aufgrund der räumlichen Distanz keinen Einfluß mehr auf die im Reaktionsgefäß befindlichen Partikel hat.

Die vertikale Lage der Arbeitsposition (B) kann verändert bzw. vorherbestimmt werden, beispielsweise in Abhängigkeit von der Länge der verwendeten Reaktionsgefäße (2) oder des verwendeten Flüssigkeitsvolumens im Reaktionsgefäß.

Der seitliche Abstand zwischen einem in Arbeitsposition befindlichen Permanentmagneten und dem zu behandelnden Reaktionsgefäß wird möglichst gering gehalten, so daß nur ein dünner Spalt von vorzugsweise weniger als 5 mm, insbesondere von weniger als 2 mm, dazwischen liegt.

Ferner ist die Vorrichtung so eingerichtet bzw. programmiert, daß die beiden Permanentmagnete (10,20) einer paarweisen Anordnung in Bezug auf das zwischen ihnen angeordnete Reaktionsgefäß (2) miteinander abwechselnd die genannte erste Position (A) oder die genannte Arbeitsposition (B) bzw. (C) einnehmen und mit vorherbestimmbarer Geschwindigkeit und Dauer zwischen den genannten Positionen wechseln.

Durch das schnelle wechselseitige Positionieren eines Magneten auf gegenüberliegenden Seiten des Reaktionsgefäßes, welches durch die erfindungsgemäßen Vorrichtungen ermöglicht wird, werden die anfänglich in einem Pellet (in Fig. 1 nicht dargestellt) enthaltenen Magnetpartikel schnell und auf effiziente Weise auseinander gerissen und nach nur wenigen Zyklen des Wechselns der Magneten resuspendiert. Dieser Effekt würde nicht - oder erst nach erheblich längerer Zeitdauer - auftreten, wenn nur ein Magnet von einer Seite des Reaktionsgefäßes auf die andere bewegt werden würde (beispielsweise indem er unter dem Reaktionsgefäß von der einen auf die andere Seite gezogen wird), weil dies erfahrungsgemäß dazu führt, daß das Pellet als Ganzes (d. h. ohne zu zerfallen) über den Boden des Reaktionsgefäßes gezogen würde. Dies wäre mit Ausbeuteverlusten, erhöhtem Zeitaufwand für die Prozessierung und einer verminderten Reinheit des Separationsprodukts verbunden.

Die erfindungsgemäßen Vorrichtungen können, je nach den Erfordernissen, so angepaßt werden, daß sie für größere Probenvolumina (z. B. im Milliliter-Bereich) oder für kleinere Probenvolumina (z. B. im Mikroliter-Bereich) optimal geeignet sind.

Die Halterung (1) kann wahlweise so ausgestaltet werden, daß sie für die Aufnahme einzelner Reaktionsgefäße, z. B. Küvetten oder Eppendorf-Gefäße, oder für die Aufnahme von Mikrotiterplatten mit einer Vielzahl von Wells geeignet ist. Ferner kann die Halterung (1) so angebracht sein, daß sie in der horizontalen Ebene in mindestens einer Richtung bewegt werden kann, vorzugsweise automatisch bzw. programmgesteuert, beispielsweise um ein Reaktionsgefäß (2) in die zwischen den Permanentmagneten (10,20) liegende Position zu bringen, oder um es zur Durchführung eines weiteren Behandlungsschrittes weiter zu transportieren.

Die einzelnen, vorstehend beschriebenen Elemente der erfindungsgemäßen Vorrichtungen können auch in mehrfacher Anzahl in einer Vorrichtung verwirklicht sein. Ferner ist nach einer weiteren Ausführungsform vorgesehen, daß mehrere der in Fig. 1 gezeigten Anordnungen von bewegbaren Permanentmagneten in einer Reihe angeordnet sind, so daß die gleichzeitige Behandlung einer Reihe von nebeneinanderliegenden Reaktionsgefäßen ermöglicht wird. Eine Vorrichtung zum magnetischen Abtrennen kann auch mehrere solcher Reihen umfassen, die jeweils mehrere Anordnungen von bewegbaren Permanentmagneten aufweisen.
Dabei kann jede der einzelnen Anordnungen über eine eigene Antriebseinheit betätigt werden, oder die mehrfach vorhandenen Anordnungen von bewegbaren Permanentmagneten können durch eine gemeinsame Antriebseinheit betätigt werden.

Die Bewegung der Permanentmagneten (10,20) wird durch eine bzw. mehrere Antriebseinheiten (4) bewirkt, gegebenenfalls unter Verwendung zusätzlicher Mittel (5) zur Kraftübertragung. Der Antrieb selbst kann mittels Elektromotoren, Elektromagneten, oder durch pneumatische oder hydraulische Mittel erfolgen, wobei die genannten Mittel auch in Kombination verwendet werden können. Der Antrieb kann z. B. eine Kurbelwelle, Exzenterscheiben, Führungsschienen, Schub- und Zuggestänge oder andere dem Fachmann bekannte Bauelemente umfassen, mittels derer die Bewegung der Magneten bewirkt wird.

Als Permanentmagnete können im Fachhandel erhältliche Magnete von geeigneter Form verwendet werden, bevorzugt handelt es sich dabei um NdFeB-Materialien. Im Einzelfall kann es vorteilhaft sein, Permanentmagnete zu verwenden, die speziell an die Form der jeweils verwendeten Reaktionsgefäße angepaßt sind. Ein Permanent-Magnet kann aus einer Vielzahl einzelner Magneten aufgebaut sein; ferner kann eine paarweise Anordnung fallweise auch mehr als zwei Permanentmagneten aufweisen.

Vorzugsweise sind die beiden Permanentmagnete (10,20) einer paarweisen Anordnung mechanisch in der Weise miteinander gekoppelt, daß der eine Magnet, bezogen auf das Reaktionsgefäß, sich in der genannten ersten Position (A) befindet, wenn der andere Magnet sich in der genannten zweiten oder anderen Position befindet (B), und umgekehrt. Des weiteren wird bevorzugt, daß die beiden Permanentmagnete (10,20) einer paarweisen Anordnung mechanisch in der Weise miteinander gekoppelt sind, daß die vertikalen Bewegungsrichtungen (a, b) der paarweise angeordneten Permanentmagneten (10,20) gegenläufig sind.
Anstelle der genannten mechanischen Kopplung kann auch eine entsprechende Schaltung oder Steuerung verwendet werden, die die gleiche Wirkung hat.

Gemäß einer weiteren bevorzugten Ausführungsform ist vorgesehen, daß der Antrieb der beweglichen Permanentmagnete programmgesteuert erfolgt. Dies kann vorzugsweise mittels einer eingebauter Mikroprozessor-Steuereinheit oder durch einen über einen Schnittstelle verbundenen Computer bewerkstelligt werden. Zusätzlich können mittels dieser Programmsteuerung auch noch weitere Funktionen der erfindungsgemäßen Vorrichtungen gesteuert werden, beispielsweise die Bewegung der Halterung (1) in horizontaler und/oder vertikaler Richtung, oder gegebenenfalls vorhandene Pipettier-Einheiten.

Vorzugsweise ist die genannte Programmsteuerung so ausgelegt, daß sie eine oder mehrere der folgenden Funktionen bzw. Parameter (einzeln oder in Kombination) der erfindungsgemäßen Vorrichtungen vorherbestimmt bzw. steuert:
- die Geschwindigkeit und/oder
- die Frequenz und/oder
- die Anzahl der Zyklen der Bewegungen der Magneten, und/oder
- die Dauer von zwischen den Bewegungen liegenden Pausenintervallen (Verweilzeit), und/oder
- die Lage der genannten ersten bzw. anderen Arbeitsposition in vertikaler Richtung, und/oder
- die Reihenfolge, in der die Permanentmagneten die genannten unterschiedlichen Positionen einnehmen.
Falls eine Vorrichtung eine Vielzahl von paarweisen Magnetanordnungen, z. B. in einer Reihe, aufweist, werden die Bewegungsabläufe dieser multiplen Einheiten vorzugsweise mittels Programmsteuerung koordiniert.

Die verschiedenen Parameter, wie Schnelligkeit der Bewegung der Permanentmagneten, Dauer bzw. Verweilzeit, Anzahl der Zyklen etc., hängen unter anderem von der Art der jeweils zu behandelnden Proben sowie von der Art des jeweiligen Behandlungsschrittes ab. Diese Parameter kann der Fachmann durch einfache Vorversuche ermitteln und optimieren.

Gemäß einer weiteren, besonders bevorzugten Ausführungsform ist vorgesehen, daß zumindest einer der bewegbaren Permanentmagneten (10, 20) in eine Position gebracht werden kann, in der er sich neben dem betreffenden Reaktionsgefäß (2) und an dessen unterem Ende befindet und in der er eine magnetische Kraft auf die Partikel ausübt (Position C in Fig. 2, Magnet (20)). Diese vorherbestimmte bzw. vorherbestimmbare Position wird "Elutionssposition" genannt, da aufgrund der Lage der Magneten am unteren Ende des Reaktionsgefäßes ein relativ geringes Volumen an Elutionsflüssigkeit ausreicht, um ein Resuspendieren der Partikel in der Elutionsflüssigkeit zu ermöglichen. Ein geringes Elutionsvolumen ist wünschenswert, damit die eluierte Zielsubstanz in möglichst hoher Konzentration erhalten wird. Umgekehrt werden bei den Waschschritten relativ große Volumina an Waschflüssigkeit eingesetzt, weshalb die obere Position des Permanentmagneten in diesem Fall vorzugsweise höher liegt, bezogen auf das Reaktionsgefäß (siehe Position B in Fig. 1).

Das Erreichen der Elutionsposition kann dadurch bewirkt werden, daß die Halterung (1) für die Reaktionsgefäße in vertikaler Richtung (Pfeil c) bewegbar angeordnet ist, und daß sie sich während des Elutionsvorgangs in einer höheren, festgelegten oder vorherbestimmbaren Position, bezogen auf das Ausgangsniveau, befindet. Das heißt, durch die Bewegung der Halterung wird der vertikale Abstand zwischen der Halterung (1) und der Permanentmagnet-Anordnung (10,20) vergrößert (Abstand y in Fig. 1 bzw. Fig. 2).
Der Abstand x zwischen der Ausgangsposition A und der Arbeitsposition B (Fig. 1) bzw. der Elutionsposition C (Fig. 2) der Permanentmagneten bleibt dabei vorzugsweise unverändert.

Alternativ können die Permanentmagnete auch dadurch in eine Elutionsposition gebracht werden, indem diese - vorzugsweise bei gleichbleibendem Niveau der Halterung (1) - unter Verkürzung der Distanz x in eine Arbeitsposition gebracht werden, die tiefer (und damit näher am Boden des Reaktionsgefäßes) liegt als die oben erwähnte Arbeitsposition, welche bei den Waschschritten verwendet wird und in Fig. 1 mit B bezeichnet ist (Magnet (20)). Auch in diesem Fall befindet sich der Permanentmagnet in der Elutionsposition neben dem betreffenden Reaktionsgefäß (2), aber an dessen unterem Ende.

Eine andere Maßnahme, mit der erreicht werden kann, daß ein Magnet in die beschriebene Elutionsposition gebracht wird, besteht darin, daß die Antriebseinheit (4) (mit der die Permanentmagnete verbunden sind) bewegt bzw. in ihrer Position verändert wird. Hierzu ist die Antriebseinheit derartig angeordnet, daß sie in vorherbestimmbarer Weise nach unten bewegt (abgesenkt) werden kann und dort während des Elutionsschrittes verbleibt. Durch eine derartige Positionierung der Antriebseinheit kann bewirkt werden, daß ein Magnet, wenn er sich in der genannten oberen oder Arbeitsstellung befindet, in der Nähe des unteren Endes des Reaktionsgefäßes zu liegen kommt. Auch in diesem Fall vergrößert sich der Abstand y bzw. der vertikale Abstand zwischen Antriebseinheit und dem unteren Ende des betreffenden Reaktionsgefäßes.

Die zuletzt beschriebene Ausführungsform der Vorrichtung, bei welcher die relative Position der Antriebseinheit veränderbar ist, ist insbesondere dann vorteilhaft, wenn eine vertikale Bewegung der Halterung (1) aus räumlichen Gründen nicht verwirklicht werden kann.
Gemäß einer bevorzugten Ausführungsform sind die Richtung und/oder Distanz und/oder Geschwindigkeit der Bewegung der Antriebseinheit bzw. der Halterung, und/oder dazwischenliegende Pausenintervalle, vorherbestimmbar oder programmierbar.

Die Bewegung der Halterung bzw. der Antriebseinheit in vertikaler und/oder horizontaler Richtung kann mit ähnlichen Mitteln erfolgen, wie dies für die Bewegung der Permanentmagnete beschrieben wurde (Motor, pneumatisch, etc.). Auch in diesem Fall ist vorzugsweise eine Programmsteuerung vorgesehen, welche es gestattet, die Richtung und/oder Distanz und/oder Geschwindigkeit der Bewegung und/oder dazwischenliegende Pausenintervalle vorherzubestimmen und zu steuern. Auch eine Koordinierung der Bewegung der Halterung bzw. der Antriebseinheit mit der Bewegung/den Bewegungen der Permanentmagnete kann auf diese Weise bewirkt werden.

Die erfindungsgemäße Vorrichtung kann ferner so ausgebildet sein, daß sie für die Permanentmagneten eine weitere Arbeitsposition vorsieht, die beispielsweise der genannten Elutionsposition annähernd entspricht und in welcher zumindest einer der Permanentmagneten befähigt ist, auf die Magnetpartikel in der Weise einzuwirken, daß diese an der Innenwand des Reaktionsgefäßes immobilisiert werden bzw. dort ein Pellet formen. Die Vorrichtung läßt sich dann auch zur Durchführung der Pelletierungsschritte des Trennverfahrens verwenden (z. B. zum Absaugen der Waschflüssigkeits-Überstände oder des die Zielsubstanz(en) enthaltenden Eluats). Abhängig vom jeweiligen Anwendungsfall kann für den Pelletierungsschritt auch eine andere, geeignete Arbeitsposition des/der Magneten gewählt werden, wie z. B. die erwähnte Arbeitsposition B in Fig. 1.
Die Verweildauer des Magneten in der genannten Position (zum Pelletieren / Immobilisieren) ist vorherbestimmbar bzw. programmierbar. Während der Verweilzeit ist der Antrieb des/der Magneten vorübergehend ausgeschaltet bzw. unterbrochen.
Der Pelletierungs/Immobilisierungs-Schritt ist üblicherweise durch eine Schaltung oder Programmsteuerung mit dem Schritt des Absaugens der Waschflüssigkeit bzw. des Eluats koordiniert bzw. synchronisiert.

Die erfindungsgemäßen Vorrichtungen können ferner auch in vorteilhafter Weise zur Durchführung des eingangs erwähnten Bindungs-Schrittes bei magnetischen Trennverfahren verwendet werden. Zu diesem Zweck ist bei der Vorrichtung vorgesehen, daß die Bewegung der Magneten (relativ zum behandelten Reaktionsgefäß) so eingestellt bzw. programmiert werden kann, daß durch diese Bewegung das gravimetrisch bedingte Sedimentieren der Magnetpartikel verhindert wird. Beispielsweise kann dies durch eine verlangsamte Bewegung der Magneten bewirkt werden.
Durch das Verhindern der gravimetrischen Sedimentation der Magnetpartikel während des Bindungs-Schrittes wird die Effizienz der Bindung der Zielsubstanz-Moleküle an die Magnetpartikel erhöht, wodurch wiederum eine Verbesserung der Ausbeute zur Folge hat.

Gemäß einer bevorzugten Ausführungsform können die erfindungsgemäßen Vorrichtungen mit Mitteln versehen sein, die für die Durchführung der weiteren Arbeitsschritte von magnetischen Trennverfahren erforderlich sind. Hierzu gehören insbesondere Vorrichtungen zum Absaugen von Flüssigkeit aus den Reaktionsgefäßen und/oder Pipettiervorrichtungen zum Hinzudosieren bzw. Absaugen definierter Flüssigkeitsvolumina zu bzw. aus den Reaktionsgefäßen. Auch diese zusätzlichen Mittel sind vorzugsweise programmsteuerbar.

Die erfindungsgemäßen Vorrichtungen können aber auch als Modul(e) in handelsübliche Liquid-Handling-Geräte eingebaut und mit diesen kombiniert werden. Das Liquid-Handling-Gerät übernimmt dabei die Vorgänge des Befüllens der Reaktionsgefäße mit Flüssigkeiten (z. B. Bindungs-, Wasch- oder Elutionsflüssigkeiten) sowie das Absaugen oder Abziehen der Flüssigkeiten aus den Reaktionsgefäßen.
Die erfindungsgemäßen Vorrichtungen übernehmen in einem solchen Kombinationsgerät einen oder mehrere der folgenden Vorgänge:
- Durchführung des Bindungs-Schrittes
- Re-Suspendieren der Magnetpartikel (z. B. in Wasch- oder Elutionsflüssigkeit)
- Pelletieren bzw. Immobilisieren der Magnetpartikel während des Absaugens/Abziehens einer Flüssigkeit.

Die erfindungsgemäße Vorrichtung und die ihr zugrunde liegende Methode der Behandlung von Magnetpartikeln zeichnen sich durch hervorragende Resuspendierungseigenschaften, durch eine hohe Effizienz beim Bindungsschritt, durch hohe Reinheiten und hohe Ausbeuten des Separationsprodukts, durch Zeitersparnis sowie durch einen geringen Verbrauch von Verbrauchsmaterial, wie Pipettenspitzen, bei der Durchführung von magnetischen Trennverfahren aus.

Durch die beschriebene Möglichkeit der Veränderung des vertikalen Abstands der Magneten (Distanz x) und/oder des vertikalen Abstands (Distanz y) zwischen den Magneten und dem Reaktionsgefäß bzw. der Halterung (1) und/oder der vertikalen Position der Antriebseinheit und/oder des horizontalen Abstands zwischen den Magneten kann die Vorrichtung auf vielfältige Weise an unterschiedlich große Reaktionsvolumina bzw. unterschiedliche Größen und Formen von Reaktionsgefäßen angepaßt werden. Aufgrund der Einfachheit des Systems ist eine gute Steuerung der einzelnen Funktionen möglich, angepaßt an die jeweilige Anwendung.

Mit der vorliegenden Erfindung wird ein Verfahren zum Behandeln von einem Magnetpartikel oder einer Vielzahl von Magnetpartikeln beschrieben, die in einem Reaktionsgefäß beispielsweise in Form eines Pellets vorliegen, um diese Partikel in einer Flüssigkeit, beispielsweise einer Wasch-, Elutions- oder Bindungsflüssigkeit, zu resuspendieren. Dies geschieht in der Weise, daß zwei paarweise angeordnete Permanentmagnete (10,20) in abwechselnder Reihenfolge auf gegenüberliegenden Seiten des Reaktionsgefäßes (2) vertikal verschoben werden, so daß sich jeweils einer der beiden Magneten (10,20) neben dem betreffenden Reaktionsgefäß befindet und eine magnetische Kraft auf die Partikel (3) ausübt und der jeweils andere der beiden Magneten (10, 20) sich in einem vertikalen Abstand zu dem Reaktionsgefäß befindet. Durch das Hin- und Herwechseln des Magneten zwischen den beiden gegenüberliegenden Seiten wird bewirkt, daß das Pellet auseinander gerissen wird und die Magnetpartikel in der Flüssigkeit suspendiert werden.

Die vorliegende Erfindung betrifft ferner Verfahren zum Behandeln von magnetisierbaren oder magnetisch anziehbaren, in einem Reaktionsgefäß, beispielsweise in Form eines Pellets, vorliegenden Partikeln mit einer Flüssigkeit, beispielsweise einer Wasch-, Elutions- oder Bindungsflüssigkeit. Diese Verfahren umfassen die Schritte:
a) Hinzufügen einer vorbestimmten Menge einer Flüssigkeit zu einem Magnetpartikel enthaltenden Pellet;
b) Resuspendieren der pelletierten Partikel und Mischen mit der Flüssigkeit,
Erfindungsgemäß wird Schritt b) dabei in der Weise durchgeführt,
- daß das genannte Reaktionsgefäß (2) zwischen zwei paarweise und gegenüberliegend angeordneten, vertikal bewegbaren Permanentmagneten (10,20) positioniert wird, und
- daß die genannte Bewegung der Permanentmagnete in der Weise erfolgt, daß jeder Permanentmagnet einer paarweisen Anordnung aus einer ersten Position oder Ausgangsposition (A), in der er sich im Abstand zu einem Reaktionsgefäß befindet und in der er im wesentlichen keinen Einfluß auf die genannten Partikel (3) ausübt, in eine zweite oder in eine andere Position (B,C) (Arbeitsposition) gebracht wird, in der er sich seitlich neben dem betreffenden Reaktionsgefäß (2) befindet und in der er eine magnetische Kraft auf die Partikel ausübt. Vorzugsweise verbleibt der Magnet jeweils für eine vorherbestimmte Zeitdauer (Verweilzeit) in dieser Position, bevor er wieder in die Ausgangsposition zurückkehrt.

Die beiden Permanentmagnete einer paarweisen Anordnung nehmen in Bezug auf das zwischen ihnen angeordnete Reaktionsgefäß miteinander abwechselnd die genannte erste Position (A) oder die genannte Arbeitsposition (B,C) ein und wechseln mit vorherbestimmbarer Geschwindigkeit und Dauer zwischen den genannten Positionen.

Durch die rasch aufeinanderfolgende abwechselnde Positionierung der Permanentmagneten auf gegenüberliegenden Seiten des Reaktionsgefäßes wird eine schnelle und effiziente Auflösung des Pellets und Resuspendierung der Magnetpartikel herbeigeführt.

Das vorstehend beschriebene Verfahren kann auch zur Durchführung des erwähnten Bindungsschrittes bei magnetischen Trennverfahren zum Einsatz kommen. Hierbei werden das/die Magnetpartikel in ein Reaktionsgefäß gegeben, welches die abzutrennende Zielsubstanz in einer Flüssigkeit (Bindungs-Flüssigkeit bzw. Bindungspuffer) enthält. Durch die beschriebene Bewegung der Permanentmagneten kann der gravitationsbedingten Sedimentation der Magnetpartikel entgegengewirkt werden, d. h. die Partikel werden während des Bindungsschrittes in der Flüssigkeit im schwebenden oder suspendierten Zustand gehalten. Infolgedessen wird die Effizienz des Bindungsschrittes gesteigert und die Ausbeute verbessert.

Damit - im Falle der Verwendung einer Elutionsflüssigkeit zum Eluieren von an den Partikeln gebundenen Substanzen - der Elutionsschritt (Schritt b) mit einem möglichst geringen Elutionsvolumen durchgeführt werden kann, wird eine Variante des Verfahren bevorzugt, bei der sich ein Permanentmagnet in der Arbeitsposition, d. h. während des Resuspendierens, seitlich neben dem betreffenden Reaktionsgefäß und an dessen unterem Ende befindet (Elutionsposition C).
Dies kann vorzugsweise dadurch erreicht werden, daß das Reaktionsgefäß oder eine das Reaktionsgefäß tragende Halterung in vertikaler Richtung, relativ zur Arbeitsposition des Permanentmagneten, aus einer unteren oder Ausgangsposition in eine vorherbestimmbare obere Position gebracht wird, wie schematisch in Fig. 2 gezeigt (Distanz y). Alternativ kann der gleiche Zweck erreicht werden, indem - wie bereits erwähnt - die Position der Antriebseinheit für die Magneten in im wesentlichen vertikaler Richtung verändert wird, oder indem die Bewegung der Permanentmagneten so gesteuert wird, daß die jeweils erreichte obere Arbeitsposition tiefer und näher am unteren Ende des Reaktionsgefäßes zu liegen kommt, verglichen mit der Arbeitsposition, die während des Resuspendierungsvorganges bei einem Wasch-Schritt erreicht wird.

Um den Resuspendierungsvorgang während eines Waschschrittes oder während Elutionsschrittes wirksamer zu gestalten, kann es vorteilhaft sein, wenn nach dem Positionieren eines Magneten in Arbeitsstellung eine Verweildauer beginnt, die in etwa so lange andauert, bis sich das Magnetpartikel-Pellet von der Gefäßwand loszulösen beginnt. Erst nach dieser Verweilzeit findet der nächste Positionierungsvorgang statt, d. h. der Wechsel zwischen Ausgangs- und Arbeitsstellung der paarweise angeordneten Permanentmagneten. Die Länge der Verweilzeit sowie die Wiederholungszyklen der Positionierungsvorgänge der Magneten werden vorzugsweise mittels eines Computerprogramms gesteuert.

Die genannten Verfahren werden vorzugsweise unter Verwendung der erfindungsgemäßen und oben beschriebenen Vorrichtungen durchgeführt.

Die erfindungsgemäßen Vorrichtungen und Verfahren lassen sich unter Ausnutzung der beschriebenen Vorteile bei den verschiedenartigen Trennverfahren, welche auf magnetisch anziehbaren Mikropartikeln beruhen, einsetzen. Aufgrund der wirksameren, schnelleren Resuspendierung kann mit der Erfindung insbesondere bei Hochdurchsatz-Verfahren eine Leistungssteigerung und Kostenersparnis erzielt werden.

### Beschreibung der Abbildungen

Fig. 1
   zeigt in Schnittdarstellung den schematischen Aufbau einer erfindungsgemäßen Vorrichtung mit einer Halterung (1) für Reaktionsgefäße (2), welche optional in Richtung des Pfeils c auf- oder abwärts bewegt werden kann. Die Halterung befindet sich im wesentlichen in einer horizontalen Ebene relativ zur Bewegungsrichtung der Permanentmagneten. Die Halterung kann so angeordnet sein, daß sie innerhalb der horizontalen Ebene in einer, zwei oder mehreren Richtungen bewegbar ist.
   Das Reaktionsgefäß (2) enthält magnetisch anziehbare bzw. magnetisierbare Partikel (Magnetpartikel) (3), üblicherweise in einer Flüssigkeit suspendiert.
   Unterhalb des Reaktionsgefäßes befindet sich eine Einheit von paarweise angeordneten Permanentmagneten (10,20), zwischen denen ein Reaktionsgefäß (2) angeordnet ist. Die Magnete werden mittels einer Antriebseinheit (4) und dazugehöriger Antriebselemente (5) in Richtung der Pfeile a bzw. b in vertikaler Richtung abwechselnd bewegt, wobei die Magnete jeweils zwischen einer Ausgangsposition (A) bzw. (B) wechseln. Im abgebildeten Fall befindet sich Magnet (10) in der Ausgangsposition (A) und Magnet (20) in Arbeitsposition (B). In der Arbeitsposition befindet sich der jeweilige Magnet neben dem Reaktionsgefäß, so daß er eine magnetische Anziehungskraft auf die im Gefäß suspendierten Partikel ausüben kann.
   Der Pfeil x bezeichnet die Distanz zwischen der Ausgangsposition und der Arbeitsposition der beiden Permanentmagneten.
   Der Pfeil y bezeichnet die Distanz zwischen der Ausgangsposition (A) der Permanentmagneten und der Halterung (1) für die Reaktionsgefäße.
Fig. 2
   entspricht Fig, 1, mit dem Unterschied, daß die Halterung (1) in vertikaler Richtung nach oben bewegt wurde, so daß die Distanz y größer ist.
   Dies hat zur Folge, daß der Permanentmagnet (20), wenn er sich in seiner Arbeitsposition C befindet, nahe dem unteren Ende des Reaktionsgefäßes zu liegen kommt. Diese Position (relativ zum Reaktionsgefäß) wird als Elutionsposition bezeichnet.
   Die Lage der Ausgangsposition A und die Länge der Distanz x ist dieselbe wie in Fig. 1.

## Patentansprüche

1. Vorrichtung zum Behandeln von magnetisierbaren oder magnetisch anziehbaren, in einer Flüssigkeit vorliegenden Partikeln (3), umfassend
- eine Halterung (1) für ein oder mehrere Reaktionsgefäß(e) (2) mit der die Partikel enthaltenden Flüssigkeit,
- mindestens zwei beweglich angeordnete Permanentmagnete (10,20),
- eine Antriebseinheit (4) zum Bewegen der Permanentmagneten (10,20);
wobei
- die Permanentmagnete (10,20) paarweise und einander gegenüberliegend angeordnet sind und in vertikaler Richtung (a, b) bewegbar sind,
- der Abstand zwischen den zwei Permanentmagneten (10,20) eines Paares so bemessen oder einstellbar ist, daß ein Reaktionsgefäß (2) zwischen diesen positioniert werden kann,
- die genannte Bewegung der Permanentmagnete in der Weise erfolgt, daß abwechselnd jeder Permanentmagnet einer paarweisen Anordnung aus einer ersten Position oder Ausgangsposition (A), in der er sich im Abstand zu einem Reaktionsgefäß (2) befindet und in der er im wesentlichen keinen Einfluß auf die genannten Partikel (3) ausübt, in eine zweite oder in eine andere Position (Arbeitsposition) (B,C) gebracht werden kann, in der er sich seitlich neben dem betreffenden Reaktionsgefäß befindet und in der er eine magnetische Kraft auf die Partikel (3) ausübt, und wobei die beiden Permanentmagnete einer paarweisen Anordnung in Bezug auf das zwischen ihnen angeordnete Reaktionsgefäß miteinander abwechselnd die genannte erste Position oder die genannte Arbeitsposition einnehmen und mit vorherbestimmbarer Geschwindigkeit und/oder Dauer zwischen den genannten Positionen wechseln.

2. Vorrichtung nach Anspruch 1, **dadurch gekennzeichnet, daß** der Antrieb der beweglichen Permanentmagnete (10,20) programmgesteuert erfolgt, vorzugsweise durch eine eingebaute Mikroprozessor-Steuereinheit oder durch einen über einen Schnittstelle verbundenen Computer.

3. Vorrichtung nach Anspruch 1 oder 2, **dadurch gekennzeichnet, daß**
- die Geschwindigkeit und/oder
- die Frequenz und/oder
- die Anzahl der Zyklen der Bewegungen der Magneten, und/oder
- die Dauer von zwischen den Bewegungen liegenden Pausenintervallen, und/oder
- die Lage der genannten ersten bzw. anderen Arbeitsposition in vertikaler Richtung, und/oder
- die Reihenfolge, in der die Permanentmagnete die genannten Positionen einnehmen vorherbestimmbar oder steuerbar, vorzugsweise durch ein Programm steuerbar ist.

4. Vorrichtung nach einem der vorangehenden Ansprüche, **dadurch gekennzeichnet, daß** die beiden Permanentmagnete (10,20) einer paarweisen Anordnung mechanisch in der Weise miteinander gekoppelt sind, daß der eine Magnet, bezogen auf das Reaktionsgefäß, sich in der genannten ersten Position (A) befindet, wenn der andere Magnet sich in der genannten zweiten oder anderen Position (B,C) befindet, und umgekehrt.

5. Vorrichtung nach einem der vorangehenden Ansprüche, **dadurch gekennzeichnet, daß** die beiden Permanentmagnete (10,20) einer paarweisen Anordnung mechanisch in der Weise miteinander gekoppelt sind, daß die vertikalen Bewegungsrichtungen der paarweise angeordneten Permanentmagneten gegenläufig sind.

6. Vorrichtung nach einem der einem der vorangehenden Ansprüche, **dadurch gekennzeichnet, daß** der Antrieb der Permanentmagnete durch Elektromotoren, durch pneumatische, hydraulische oder elektromagnetische Antriebsmittel, oder durch eine Kombination mindestens zweier der genannten Mittel erfolgt.

7. Vorrichtung nach einem der einem der vorangehenden Ansprüche, **dadurch gekennzeichnet, daß** sie eine Vielzahl der genannten paarweise angeordneten Permanentmagneten (10,20) aufweist, vorzugsweise in reihenförmiger Anordnung.

8. Vorrichtung nach einem der einem der vorangehenden Ansprüche, **dadurch gekennzeichnet, daß** eine weitere Position (genannt Elutions-Position; (C)) vorgesehen ist, in der sich der Permanentmagnet neben dem betreffenden Reaktionsgefäß und an dessen unterem Ende befindet und in der er eine magnetische Kraft auf die Partikel (3) ausübt.

9. Vorrichtung nach einem der vorangehenden Ansprüche, **dadurch gekennzeichnet, daß** die genannte Halterung (1) in vertikaler Richtung (c) bewegbar angebracht ist und durch Antriebsmittel aus einer unteren oder Ausgangsposition in eine vorherbestimmbare obere Position gebracht werden kann, so daß ein Permanentmagnet (10,20), wenn er sich in der genannten Arbeitsposition an einem Reaktionsgefäß befindet, am unteren Ende des jeweiligen Reaktionsgefäßes positioniert ist und sich in der Elutions-Position (C) befindet.

10. Vorrichtung nach einem der vorangehenden Ansprüche, **dadurch gekennzeichnet, daß** die genannte Antriebseinheit (4) bewegbar angebracht ist und durch Antriebsmittel aus einer oberen oder Ausgangsposition in eine vorherbestimmbare untere Position gebracht werden kann, so daß ein Permanentmagnet (10,20), wenn er sich in der genannten Arbeitsposition (B,C) an einem Reaktionsgefäß (2) befindet, am unteren Ende des jeweiligen Reaktionsgefäßes (2) positioniert ist,
wobei bevorzugt wird, daß die Richtung und/oder Distanz und/oder Geschwindigkeit der Bewegung der Antriebseinheit und/oder dazwischenliegende Pausenintervalle vorherbestimmbar oder programmierbar sind.

11. Vorrichtung nach einem der vorangehenden Ansprüche, **dadurch gekennzeichnet, daß** die genannte Halterung (1) in horizontaler und/oder vertikaler Richtung bewegbar angebracht ist, wobei bevorzugt wird, daß die Richtung und/oder Distanz und/oder Geschwindigkeit der Bewegung und/oder dazwischenliegende Pausenintervalle vorherbestimmbar oder programmierbar sind.

12. Vorrichtung nach einem der vorangehenden Ansprüche, **dadurch gekennzeichnet, daß** sie Vorrichtungen zum Absaugen von Flüssigkeit aus den Reaktionsgefäßen (2) und/oder Pipettiervorrichtungen zum Hinzudosieren definierter Flüssigkeitsvolumina zu den Reaktionsgefäßen aufweist, wobei diese Arbeitsschritte vorzugsweise programmsteuerbar sind.

13. Verfahren zum Behandeln von magnetisierbaren oder magnetisch anziehbaren, in einem Reaktionsgefäß (2) vorliegenden Partikeln (3), um die Partikel in einer hinzugegebenen Flüssigkeit zu resuspendieren, **dadurch gekennzeichnet, daß** zwei paarweise angeordnete Permanentmagnete (10,20) in abwechselnder Reihenfolge auf gegenüberliegenden Seiten des Reaktionsgefäßes (2) vertikal verschoben werden, so daß sich jeweils einer der beiden Magneten (10,20) neben dem betreffenden Reaktionsgefäß befindet und eine magnetische Kraft auf die Partikel (3) ausübt und der jeweils andere der beiden Magneten (10, 20) sich in einem vertikalen Abstand zu dem Reaktionsgefäß befindet.

14. Verfahren zum Behandeln von magnetisierbaren oder magnetisch anziehbaren, in einem Reaktionsgefäß vorliegenden Partikeln (3) mit einer Flüssigkeit, umfassend die Schritte:
a) Hinzufügen einer vorbestimmten Menge einer Flüssigkeit zu einem Magnetpartikel (3) enthaltenden Pellet;
b) Resuspendieren der pelletierten Partikel und Mischen mit der Flüssigkeit,
**dadurch gekennzeichnet, daß** Schritt b) in der Weise durchgeführt wird,
- daß das genannte Reaktionsgefäß zwischen zwei paarweise und gegenüberliegend angeordneten, vertikal bewegbaren Permanentmagneten (10,20) positioniert wird, und
- daß die genannte Bewegung der Permanentmagnete (10,20) in der Weise erfolgt, daß abwechselnd jeder Permanentmagnet einer paarweisen Anordnung aus einer ersten Position oder Ausgangsposition (A), in der er sich im Abstand zu einem Reaktionsgefäß (2) befindet und in der er im wesentlichen keinen Einfluß auf die genannten Partikel (3) ausübt, in eine zweite oder in eine andere Position (Arbeitsposition) (B,C) gebracht wird, in der er sich seitlich neben dem betreffenden Reaktionsgefäß befindet und in der er eine magnetische Kraft auf die Partikel (3) ausübt, und
wobei die beiden Permanentmagnete einer paarweisen Anordnung in Bezug auf das zwischen ihnen angeordnete Reaktionsgefäß miteinander abwechselnd die genannte erste Position oder die genannte Arbeitsposition einnehmen und mit vorherbestimmbarer Geschwindigkeit und Dauer zwischen den genannten Positionen wechseln.

15. Verfahren nach Anspruch 14, **dadurch gekennzeichnet, daß** als Flüssigkeit eine Bindungs-Flüssigkeit, WaschFlüssigkeit oder Elutions-Flüssigkeit verwendet wird zur Durchführung der bei magnetischen Trennverfahren vorgesehenen Bindungs-, Wasch- oder Elutionsschritte.

16. Verfahren nach Anspruch 14 oder 15, **dadurch gekennzeichnet, daß** Schritt b) in der Weise durchgeführt wird, daß
- daß das genannte Reaktionsgefäß (2) zwischen zwei paarweise und gegenüberliegend angeordneten, vertikal bewegbaren Permanentmagneten (20,30) positioniert wird, und
- daß jeder Permanentmagnet der genannten paarweisen Anordnung abwechselnd in eine Elutions-Position (C) gebracht wird, in der er sich seitlich neben dem betreffenden Reaktionsgefäß und an dessen unterem Ende befindet und in der er eine magnetische Kraft auf die Partikel ausübt.

17. Verfahren nach Anspruch 16, **dadurch gekennzeichnet, daß** der genannte Permanentmagnet **dadurch** in die beschriebene Elutions-Position (C) gebracht wird,
- indem das Reaktionsgefäß oder eine das Reaktionsgefäß tragende Halterung (1) in vertikaler Richtung (c), relativ zur Arbeitsposition (B) des Permanentmagneten, aus einer unteren oder Ausgangsposition in eine vorherbestimmbare obere Position gebracht wird, oder/und
- indem die genannte Ausgangsposition (A) der Permanentmagneten in eine vorherbestimmbare untere Position gebracht wird,
so daß ein Permanentmagnet, wenn er sich in der genannten Arbeitsposition (B) an einem Reaktionsgefäß befindet, am unteren Ende des jeweiligen Reaktionsgefäßes positioniert ist.

18. Verfahren nach einem der Ansprüche 13 bis 17, **dadurch gekennzeichnet, daß** die genannten Bewegungen der Permanentmagneten (10,20) bzw. der Reaktionsgefäß-Halterung (1) automatisch und/oder programmgesteuert durchgeführt werden.

19. Verfahren einem der Ansprüche 13 bis 18, **dadurch gekennzeichnet, daß** der Schritt des Resuspendierens in der Weise erfolgt, daß auf jede Positionsänderung der Magneten eine Verweilzeit folgt, deren Länge vorherbestimmt ist.

20. Verfahren nach einem der Ansprüche 13 bis 19, **dadurch gekennzeichnet, daß** es unter Verwendung einer Vorrichtung nach einem oder mehreren der Ansprüche 1 bis 12 durchgeführt wird.

21. Verwendung einer Vorrichtung nach einem oder mehreren der Ansprüche 1 bis 12 bei der Abtrennung von Substanzen mittels magnetisierbarer oder magnetisch anziehbarer Partikel,
(a) zur Durchführung des Bindungsschrittes und/oder
(b) zum Resuspendieren und/oder
(c) bei einem Wasch-Schritt und/oder
(d) zum Pelletieren/Immobilisieren der Partikel und/oder
(e) bei einem Elutions-Schritt.

## Claims

1. Device for treating magnetizable or magnetically attractable particles (3) which are present in a liquid, comprising
- a holder (1) for holding one ore more reaction vessel(s) (2) with the liquid containing the particles,
- at least two movably arranged permanent magnets (10, 20),
- a drive unit (4) for moving the permanent magnets (10, 20);
with
- the permanent magnets (10, 20) being arranged in pairs and opposite to each other, and being movable in vertical direction (a, b),
- the distance between the two permanent magnets (10, 20) of a pair being dimensioned or adjustable such that a reaction vessel (2) can be positioned therebetween,
- the aforementioned movement of the permanent magnets taking place in such a way that each permanent magnet of a paired arrangement can alternately be brought from a first position or start position (A), in which it is at a distance from the reaction vessel (2) and has essentially no influence on the mentioned particles (3), to a second or to another position (work position) (B, C), in which it is located laterally next to the reaction vessel concerned and exerts a magnetic force on the particles (3),
and with the two permanent magnets of a paired arrangement alternating with each other in taking over the above-mentioned first position or the above-mentioned work position, relative to the reaction vessel positioned between the magnets, and alternating between the said positions at a predetermined velocity and/or for a predetermined duration.

2. Device according to Claim 1, **characterized in that** the drive of the movable permanent magnets (10, 20) is programme-controlled, preferably by means of an integrated microprocessor control unit or by means of a computer connected via an interface.

3. Device according to Claim 1 or 2, **characterized in that**
- the velocity, and/or
- the frequency, and/or
- the number of cycles of the movements of the magnets, and/or
- the duration of the pause intervals between the movements,
and /or
- the position of the said first or other working position in vertical direction, and/or
- the sequence in which the permanent magnets take up the above-mentioned positions
is/are predeterminable or controllable, preferably by means of a programme.

4. Device according to any one of the preceding claims, **characterized in that** the two permanent magnets (10, 20) of a paired arrangement are mechanically coupled to each other in such a way that, relative to the reaction vessel, the one magnet is located in the said first position (A) when the other magnet is located in the said second or other position (B, C), and vice versa.

5. Device according to any one of the preceding claims, **characterized in that** the two permanent magnets (10, 20) of a paired arrangement are coupled to each other in such a manner that the vertical directions of movement of the pair-wise arranged permanent magnets are opposite to each other.

6. Device according to any one of the preceding claims, **characterized in that** the drive of the permanent magnets is accomplished by electro-motors, by pneumatic, hydraulic or electromagnetic drive means, or by a combination of at least two of the above-mentioned means.

7. Device according to any one of the preceding claims, **characterized in that** it comprises a plurality of the said pair-wise arranged permanent magnets (10, 20), preferably arranged in a row.

8. Device according to any one of the preceding claims, **characterized in that** a further position (called elution position; (c)) is provided, in which the permanent magnet is located beside the reaction vessel concerned and at the bottom end thereof, and in which it exerts a magnetic force on the particles (3).

9. Device according to any one of the preceding claims, **characterized in that** the said holder (1) is arranged so as to be movable in vertical direction (c), and can be brought from a lower or starting position to a predeterminable upper position by drive means, so that a permanent magnet (10, 20), when located in the said working position at a reaction vessel, is positioned at the bottom end of the reaction vessel concerned and is located in the elution position (C).

10. Device according to any one of the preceding claims, **characterized in that** the said drive unit (4) is movably arranged and can be brought from an upper or starting position to a predeterminable lower position by drive means, so that a permanent magnet (10, 20), when located in the said working position (B, C) at a reaction vessel (2), is positioned at the bottom end of the respective reaction vessel (2), it being preferred that the direction and/or distance and/or velocity of the movement of the drive unit, and/or intermediate pause intervals are predeterminable or programmable.

11. Device according to any one of the preceding claims, **characterized in that** the said holder (1) is arranged so as to be movable in horizontal and/or vertical direction, it being preferred that the direction and/or distance and/or velocity of the movement, and/or the intermediate pause intervals are predeterminable or programmable.

12. Device according to any one of the preceding claims, **characterized in that** it comprises devices for sucking-off of liquid from the reaction vessels (2) and/or pipetting devices for dosing defined volumes of liquid to the reaction vessels, these operational steps preferably being programme-controllable.

13. Process for treating magnetizable or magnetically attractable particles (3), which are present in a reaction vessel (2), in order to resuspend the particles in a liquid to be added thereto, **characterized in that** two permanent magnets (10, 20), which are arranged in a pair, are vertically displaced in alternating order at opposite sides of the reaction vessel (2), so that in each case one of the two magnets (10, 20) is located beside the reaction vessel concerned and exerts a magnetic force on the particles (3), and **in that** the respective other of the two magnets (10, 20) is located at a vertical distance to the reaction vessel.

14. Process for treating magnetizable or magnetically attractable particles (3), which are present in a reaction vessel, with a liquid, said process comprising:
a) adding a predetermined amount of a liquid to a pellet containing magnetic particles (3);
b) resuspending the pelletized particles and mixing with the liquid,
**characterized in that** step b) is carried out in such a manner that:
- the said reaction vessel is positioned between two permanent magnets (10, 20) which are arranged in a pair and opposite to each other, and are vertically movable, and
- the said movement of the permanent magnets (10, 20) is performed such that alternately each permanent magnet of a paired arrangement is brought from a first position or starting position (A), wherein it is located at a distance from a reaction vessel (2) and wherein it has substantially no influence on the said particles (3), to a second or to another position (working position) (B, C), wherein it is located laterally next to the reaction vessel concerned and wherein it exerts a magnetic force on the particles (3), with the two permanent magnets of a paired arrangement alternating with each other in taking over the said first position or the said working position relative to the reaction vessel arranged between the magnets, and changing between the said positions at a predeterminable velocity and for predeterminable durations.

15. Process according to Claim 14, **characterized in that** a binding liquid, wash liquid or elution liquid is used as the said liquid for carrying out the binding, washing or elution step in magnetic separation processes.

16. Process according to claim 14 or 15, **characterized in that** step b) is carried out in such a manner that
- the said reaction vessel (2) is positioned between two vertically movable permanent magnets (20, 30) which are arranged in a pair and opposite to each other, and
- that each permanent magnet of the said paired arrangement is alternately brought into an elution position (C), in which it is located laterally next to the reaction vessel in question and at the bottom end thereof, and in which it exerts a magnetic force on the particles.

17. Process according to claim 16, **characterized in that** the said permanent magnet is brought to the described elution position (c) by
- moving the reaction vessel or a holder (1) carrying the reaction vessel in vertical direction (c) from a lower or starting position to a predeterminable upper position, relative to the working position (B) of the permanent magnet,
- moving the said starting position (A) of the permanent magnets to a predeterminable lower position,
so that a permanent magnet, when located in the said work position (B) at a reaction vessel, is positioned at the bottom end of the reaction vessel concerned.

18. Process according to any one of claims 13 to 17, **characterized in that** the said movements of the permanent magnets (10, 20), or respectively of the reaction vessel holder (1), are carried out automatically and/or programme-controlled.

19. Process according to any one of claims 13 to 18, **characterized in that** the resuspension step is carried out in such a manner that each altering of the position of the magnets is followed by a dwell time the length of which is predetermined.

20. Process according to any of claims 13 to 19, **characterized in that** it is carried out utilizing a device according to one or more of claims 1 to 12.

21. Use of a device according to one or more of claims 1 to 12 in separating substances by means of magnetizable or magnetically attractable particles
(a) for carrying out the binding step, and/or
(b) for resuspending, and/or
(c) in a washing step, and/or
(d) for pelletizing/immobilizing the particles, and/or
(e) in an elution step.

## Revendications

1. Dispositif de traitement de particules (3) magnétisables ou pouvant être excitées magnétiquement, présentes dans un liquide, comprenant
- un support (1) pour un ou plusieurs vases à réaction (2) avec le liquide contenant les particules,
- au moins deux aimants permanents disposés de façon mobile (10, 20),
- une unité de mise en marche (4) pour déplacer les aimants permanents (10, 20),
dans lequel
- les aimants permanents (10, 20) sont disposés par paires et se faisant face, et étant mobiles dans le sens vertical (a, b),
- l'intervalle entre les deux aimants permanents (10, 20) d'une paire est mesuré ou réglable de manière que l'on puisse positionner un vase à réaction (2) entre eux,
- le mouvement mentionné des aimants permanents s'effectue de manière qu'alternativement chaque aimant permanent d'une disposition par paires puisse être amené à partir d'une première position ou position de départ (A) dans laquelle il se trouve à un certain intervalle d'un vase à réaction (2) et dans laquelle il n'exerce pour l'essentiel aucune influence sur les particules (3) mentionnées, en une seconde ou autre position (position de travail) (B, C) dans laquelle il se trouve latéralement proche du vase à réaction en question et dans laquelle il exerce une force magnétique sur les particules (3), et dans lequel les deux aimants permanents d'une disposition par paires prennent par rapport au vase à réaction disposé entre eux, de façon alternée, la première position mentionnée ou la position de travail mentionnée et se relayent selon une vitesse et/ou une durée que l'on peut déterminer à l'avance entre les positions mentionnées.

2. Dispositif selon la revendication 1, **caractérisé ce que** la mise en marche des aimants permanents mobiles (10, 20) s'effectue d'une manière réglée par un programme, de préférence au moyen d'une unité de réglage à microprocesseur intégrée, ou au moyen d'un ordinateur relié par l'intermédiaire d'un point de coupure.

3. Dispositif selon la revendication 1 ou 2, **caractérisé en ce que**
- la vitesse et/ou
- la fréquence et/ou
- le nombre de cycles des mouvements des aimants, et/ou
- la durée des intervalles de pause qui interviennent entre les mouvements, et/ou
- la situation de la première position mentionnée, resp. de l'autre position de travail sur l'axe vertical, et/ou
- la succession selon laquelle les aimants permanents prennent les positions mentionnées
sont prédéterminables à l'avance ou réglables, de préférence réglables au moyen d'un programme.

4. Dispositif selon l'une des revendications précédentes, **caractérisé en ce que** les deux aimants permanents (10, 20) d'une disposition par paires sont couplés entre eux mécaniquement de manière qu'un aimant, par rapport au vase à réaction, se trouve dans la première position (A) mentionnée lorsque l'autre aimant se trouve dans la seconde ou autre position mentionnée (B, C), et inversement.

5. Dispositif selon l'une des revendications précédentes, **caractérisé en ce que** les deux aimants permanents (10, 20) d'une disposition par paires sont couplés mécaniquement entre eux de manière que les sens de mouvement verticaux des aimants permanents disposés par paires soient opposés.

6. Dispositif selon l'une des revendications précédentes, **caractérisé en ce que** la mise en marche des aimants permanents s'effectue par des moteurs électriques, par des moyens de mise en marche pneumatiques, hydrauliques ou électromagnétiques, ou par une combinaison d'au moins deux des moyens mentionnés.

7. Dispositif selon l'une des revendications précédentes, **caractérisé en ce qu'**il présente une pluralité des aimants permanents disposés par paires mentionnés (10, 20), de préférence en une disposition en forme de rangée.

8. Dispositif selon l'une des revendications précédentes, **caractérisé en ce qu'**est prévue une autre position (dite position d'élution ; (C), dans laquelle l'aimant permanent se trouve à côté du vase à réaction en question et à son extrémité inférieure, et dans laquelle il exerce une force magnétique sur les particules (3).

9. Dispositif selon l'une des revendications précédentes, **caractérisé en ce que** le support (1) mentionné est introduit de manière à être mobile dans le sens vertical (c) et peut être amené, par des moyens de mise en marche, d'une position inférieure ou de départ à une position supérieure prédéterminable, si bien qu'un aimant permanent (10, 20), lorsqu'il se trouve dans la position de travail mentionnée sur un vase à réaction, est positionné à la partie inférieure du vase à réaction considéré et se trouve dans la position d'élution (C).

10. Dispositif selon l'une des revendications précédentes, **caractérisé en ce que** l'unité de mise en marche (4) mentionnée est introduite de manière à pouvoir être déplacée, et peut être amenée, par des moyens de mise en marche, d'une position supérieure ou de départ à une position inférieure qui peut être prédéterminée, si bien qu'un aimant permanent (10, 20), lorsqu'il se trouve dans la position de travail (B, C) mentionnée sur un vase à réaction (2), est positionné à l'extrémité inférieure du vase à réaction (2) en question, où l'on préfère que le sens et/ou la distance et/ou la vitesse du mouvement de l'unité de mise en marche et/ou les intervalles de pause intermédiaires soient prédéterminables à l'avance ou programmables.

11. Dispositif selon l'une des revendications précédentes, **caractérisé en ce que** le support (1) mentionné est introduit de manière à pouvoir se déplacer dans le sens horizontal et/ou vertical, où l'on préfère que le sens et/ou la distance et/ou la vitesse du déplacement et/ou les intervalles de pause intermédiaires soient prédéterminables à l'avance ou programmables.

12. Dispositif selon l'une des revendications précédentes, **caractérisé en ce qu'**il présente des dispositifs pour aspirer le liquide des vases à réaction (2) et/ou des dispositifs d'introduction avec une pipette pour doser des volumes de liquide définis dans les vases à réaction, cette étape de travail étant de préférence réglable au moyen d'un programme.

13. Procédé de traitement de particules (3) magnétisables ou pouvant être excitées par un moyen magnétique, présentes dans un vase à réaction (2), pour remettre en suspension les particules dans un liquide à ajouter, **caractérisé en ce qu'**on déplace verticalement deux aimants permanents (10, 20) disposés par paires, selon une succession alternée, sur des côtés opposés du vase à réaction (2), si bien qu'un des deux aimants (10, 20) se trouve toujours à côté du vase à réaction en question et exerce une force magnétique sur les particules (3), et l'autre des deux aimants (10, 20) se trouve toujours à un certain intervalle vertical par rapport au vase à réaction.

14. Procédé de traitement de particules (3) magnétisables ou pouvant être excitées par un moyen magnétique, présentes dans un vase à réaction (3) avec un liquide comprenant les étapes suivantes :
a) addition d'une quantité prédéterminée d'un liquide à un précipité contenant des particules magnétiques (3) ;
b) remise en suspension des particules précipitées et mélange avec le liquide ;
**caractérisé en ce qu'**on conduit l'étape b) de manière
- que le vase à réaction mentionné soit positionné entre deux aimants permanents (10, 20) disposés par paires et se faisant face, pouvant se déplacer dans le sens vertical, et
- que le mouvement mentionné des aimants permanents (10, 20) s'effectue de manière qu'alternativement chaque aimant permanent d'une disposition par paires soit amené d'une première position ou position de départ (A), dans laquelle il se trouve à un certain intervalle d'un vase à réaction (2) et dans laquelle il n'exerce pour l'essentiel aucune influence sur les particules (3) mentionnées, à une seconde ou autre position (position de travail) (B, C) dans laquelle il se trouve latéralement à côté du vase à réaction en question et dans laquelle il exerce une force magnétique sur les particules (3), et où les deux aimants permanents d'une disposition par paires prennent alternativement entre eux, par rapport au vase à réaction disposé entre eux, la première position mentionnée ou la position de travail mentionnée et se relayent selon une vitesse et une durée que l'on peut déterminer à l'avance entre les deux positions mentionnées.

15. Procédé selon la revendication 14, **caractérisé en ce qu'**on utilise comme liquide un liquide de liaison, un liquide de lavage ou un liquide d'élution pour la réalisation des étapes de liaison, de lavage ou d'élution prévues dans le procédé de séparation magnétique.

16. Procédé selon la revendication 14 ou 15, **caractérisé en ce qu'**on réalise l'étape b) de manière
- que le vase à réaction (2) mentionné soit positionné entre deux aimants permanents (20, 30) disposés par paires et se faisant face, pouvant se déplacer verticalement, et
- que chaque aimant permanent de la disposition par paires mentionnée soit amené alternativement dans une position d'élution (C) dans laquelle il se trouve latéralement à côté du vase à réaction en question et à son extrémité inférieure et dans laquelle il exerce une force magnétique sur les particules.

17. Procédé selon la revendication 16, **caractérisé en ce que** l'aimant permanent mentionné est amené dans la position d'élution (C) décrite
- en amenant le vase à réaction ou un support (1) portant le vase à réaction dans le sens vertical (c), relativement à la position de travail (B) de l'aimant permanent, d'une position inférieure ou de départ, à une position supérieure que l'on peut prédéterminer à l'avance, et/ou
- en amenant la position de départ (A) mentionnée des aimants permanents, à une position inférieure que l'on peut prédéterminer à l'avance,
si bien qu'un aimant permanent, lorsqu'il se trouve dans la position de travail (B) mentionnée sur un vase à réaction, est positionné à l'extrémité inférieure du vase à réaction en question.

18. Procédé selon l'une des revendications 13 à 17, **caractérisé en ce que** les déplacements mentionnés des aimants permanents (10, 20), resp. du support (1) de vase à réaction sont effectués de manière automatique et/ou réglée par un programme.

19. Procédé selon l'une des revendications 13 à 18, **caractérisé en ce que** l'étape de remise en suspension s'effectue de manière que chaque modification de position des aimants est suivie par un temps d'arrêt dont la longueur est prédéterminée à l'avance.

20. Procédé selon l'une des revendications 13 à 19, **caractérisé en ce qu'**on procède en utilisant un dispositif selon une ou plusieurs des revendications 1 à 12.

21. Utilisation d'un dispositif selon une ou plusieurs des revendications 1 à 12 dans la séparation de substances au moyen de particules magnétisables ou pouvant être excitées par un moyen magnétique,
(a) pour réaliser l'étape de liaison et/ou
(b) pour la remise en suspension et/ou
(c) dans une étape de lavage et/ou
(d) pour la précipitation/immobilisation des particules et/ou
(e) dans une étape d'élution.
